Europäisches Patentamt
European Patent Office
Office européen des brevets

(11) Publication number: 0 295 118 A1

# EUROPEAN PATENT APPLICATION

(21) Application number: 88305307.6

(22) Date of filing: 10.06.88

(51) Int. Cl.4: C 07 D 231/16, C 07 D 231/36, C 07 D 231/40, C 07 D 403/04, A 01 N 43/56

(30) Priority: 12.06.87 GB 8713769

(43) Date of publication of application: 14.12.88 Bulletin 88/50

(84) Designated Contracting States: AT BE CH DE ES FR GB GR IT LI LU NL SE

(71) Applicant: MAY & BAKER LIMITED
Dagenham Essex RM10 7XS (GB)

(72) Inventor: Buntain, Ian George
May&Baker Limited
Dagenham Essex RM10 7XS (GB)

Hatton, Leslie Roy
May&Baker Limited
Dagenham Essex RM10 7XS (GB)

Hawkins, David William
May&Baker Limited
Dagenham Essex RM10 7XS (GB)

Pearson, Christopher John
May&Baker Limited
Dagenham Essex RM10 7XS (GB)

Roberts, David Alan
May&Baker Limited
Dagenham, Essex, RM10 7XS (GB)

(74) Representative: Bentham, Stephen et al
J.A. Kemp & Co. 14 South Square Gray's Inn
London WC1R 5EU (GB)

## (54) N-phenylpyrazole derivatives.

(57) 4-Nitro-N-phenylpyrazole derivatives of the formula:

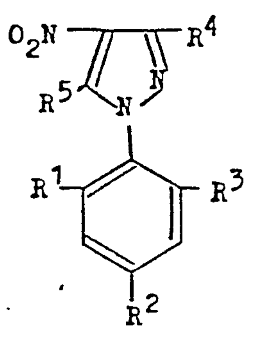

I

wherein $R^1$ represents fluorine, chlorine or bromine, $R^2$ represents chlorine or bromine, or alkyl or alkoxy group optionally substituted by halogen, $R^3$ is as defined for $R^1$ or represents hydrogen, $R^4$ represents halogen, cyano or nitro, $R^5$ represents hydrogen, an amino group $-NR^6R^7$ wherein $R^6$ and $R^7$, each represents hydrogen, alkyl, formyl, alkanoyl (or $R^6$ and $R^7$ together form a cyclic imide) which may be unsubstituted or substituted by halogen, alkoxycarbonyl which may be unsubstituted or substituted by halogen, or $R^5$ represents alkoxymethyleneamino which may be unsubstituted or substituted on methylene by alkyl, or represents fluorine, chlorine, bromine or iodine possess pesticidal properties; their preparation and use, and compositions containing them are described.

EP 0 295 118 A1

## Description

# N-PHENYLPYRAZOLE DERIVATIVES

This invention relates to 4-nitro-N-phenylpyrazole derivatives, to compositions containing them and to the use of 4-nitro-N-phenylpyrazole derivatives against arthropod, plant nematode, helminth and protozoan pests.

The present invention provides 4-nitro-N-phenylpyrazole derivatives of the general formula I depicted hereinafter wherein $R^1$ represents a fluorine, chlorine or bromine atom, $R^2$ represents a chlorine or bromine atom, or a straight- or branched-chain alkyl or alkoxy group containing from 1 to 4 carbon atoms which may be unsubstituted or substituted by one or more halogen atoms, (e.g. a trifluoromethyl or trifluoromethoxy group), $R^3$ is as defined for $R^1$ or represents the hydrogen atom, $R^4$ represents a halogen, i.e. fluorine, chlorine, bromine or iodine, atom, or the cyano or nitro group, $R^5$ represents the hydrogen atom, or the amino group $-NR^6R^7$ wherein $R^6$ and $R^7$, which may be the same or different, each represents the hydrogen atom or a straight- or branched-chain alkyl group containing from 1 to 6 carbon atoms, the formyl group, a straight- or branched-chain alkanoyl group containing from 2 to 7 carbon atoms (or $R^6$ and $R^7$ together form a 5 or 6 membered cyclic imide with the nitrogen atom to which they are attached) which may be unsubstituted or substituted by one or more halogen atoms, or a straight- or branched-chain alkoxycarbonyl group containing from 2 to 7 carbon atoms, which may be unsubstituted or substituted by one or more halogen atoms, or $R^5$ represents a straight- or branched-chain alkoxymethyleneamino group containing from 2 to 5 carbon atoms which may be unsubstituted or substituted on methylene by a straight- or branched-chain alkyl group containing from 1 to 4 carbon atoms, or represents a fluorine, chlorine ,bromine or iodine atom, have valuable activity against arthropod, plant nematode, helminth and protozoan pests, more particularly by ingestion of the compound(s) of general formula I by the arthropods.

When groups are substituted by more than one halogen atom it is to be understood that the halogen atoms may be the same or different.

Preferred compounds of general formula I are those wherein $R^1$, $R^2$ and $R^3$ together represent 2,4,6-trichloro, 2,6-dichloro-4-trifluoromethyl or 2,6-dichloro-4-trifluoromethoxy substitution of the phenyl group.

Preferred compounds are those wherein $R^5$ represents the hydrogen atom or $-NR^6R^7$, preferably an amino or acetamido group, $R^1$, $R^2$ and $R^3$ together represent 2,6-dichloro-4-trifluoromethyl or 2,6-dichloro-4-trifluoromethoxy substitution of the phenyl group, and $R^4$ is as hereinbefore defined.

The following compounds of general formula I are of particular interest as insecticides:-

1. 5-Acetamido-3-bromo-l-(2,6-dichloro-4-trifluoromethylphenyl)-4-nitropyrazole.
2. 5-Acetamido-3-chloro-l-(2,6-dichloro-4-trifluoromethylphenyl)-4-nitropyrazole.
3. 5-Acetamido-3-cyano-l-(2,6-dichloro-4-trifluoromethylphenyl)-4-nitropyrazole.
4. 5-Amino-3-bromo-l-(2,6-dichloro-4-trifluoromethylphenyl)-4-nitropyrazole.
5. 5-Amino-3-chloro-l-(2,6-dichloro-4-trifluoromethylphenyl)-4-nitropyrazole.
6. 5-Amino-3-cyano-l-(2,6-dichloro-4-trifluoromethylphenyl)-4-nitropyrazole.
7. 5-Bromo-3-cyano-1-(2,6-dichloro-4-trifluoromethylphenyl)-4-nitropyrazole.

According to a feature of the present invention, there is provided a method for the control of arthropod, plant nematode, helminth or protozoan pests at a locus which comprises the treatment of the locus (e.g. by application or administration) with an effective amount of a compound of general formula (I), wherein the various symbols are as hereinbefore defined. The compounds of general formula (I) may, in particular, be used in the field of veterinary medicine and livestock husbandry and in the maintenance of public health against arthropods, helminths or protozoa which are parasitic internally or externally upon vertebrates, particularly warm-blooded vertebrates, for example man and domestic animals, e.g. cattle, sheep, goats, equines, swine, poultry, dogs, cats and fishes, for example Acarina, including ticks (e.g. Ixodes spp., Boophilus spp. e.g. Boophilus microplus, Amblyomma spp., Hyalomma spp., Rhipicephalus spp. e.g. Rhipicephalus appendiculatus, Haemaphysalis spp., Dermacentor spp., Ornithodorus spp. (e.g. Ornithodorus moubata and mites (e.g. Damalinia spp., Dermahyssus gallinae, Sarcoptes spp. e.g. Sarcoptes scabiei, Psoroptes spp., Chorioptes spp., Demodex spp., Eutrombicula spp.,); Diptera (e.g. Aedes spp., Anopheles spp., Musca spp., Hypoderma spp., Gasterophilus spp., Simulium spp.); Hemiptera (e.g. Triatoma spp.); Phthiraptera (e.g. Damalinia spp., Linognathus spp.); Siphonaptera (e.g. Ctenocephalides spp.); Dictyoptera (e.g. Periplaneta spp., Blatella spp.); Hymenoptera (e.g. Monomorium pharaonis); for example against infections of the gastro-intestinal tract caused by parasitic nematode worms, for example members of the family Trichostrongylidae, Nippostrongylus brasiliensis, Trichinella spiralis, Haemonchus contortus, Trichostrongylus colubriformis, Nematodirus battus, Ostertergia circumcincta, Trichostrongylus axei, Cooperia spp. and Hymenolepis nana; in the control and treatment of protozoal diseases caused by, for example, Eimeria spp. e.g. Eimeria tenella, Eimeria acervulina, Eimeria brunetti, Eimeria maxima and Eimeria necatrix, Trypanosoma cruzi, Leishmania spp., Plasmodium spp., Babesia spp., Trichomonadidae spp., Histomonas spp., Giardia spp., Toxoplasma spp., Entamoeba histolytica and Theileria spp.; in the protection of stored products, for example cereals, including grain and flour, groundnuts, animal feedstuffs, timber and household goods, e.g. carpets and textiles, against attack by arthropods, more especially beetles, including weevils, moths and mites, for example Ephestia spp. (flour moths), Anthrenus spp. (carpet beetles), Tribolium spp. (flour beetles), Sitophilus spp. (grain weevils) and Acarus spp. (mites), in the control of cockroaches, ants and termites and similar arthropod pests in infested

domestic and industrial premises and in the control of mosquito larvae in waterways, wells, reservoirs or other running or standing water; for the treatment of foundations,structure and soil in the prevention of the attack on buildings by termites, for example Reticulitermes spp.,Heterotermes spp., Coptotermes spp.; in agriculture, against adults, larvae and eggs of Lepidoptera (butterflies and moths), e.g. Heliothis spp. such as Heliothis virescens (tobacco budworm), Heliothis armigera and Heliothis zea, Spodoptera spp. such as S.exempta S.littoralis (Egyptian cotton worm), S.eridania (southern army worm), Mamestra configurata (bertha army worm); Earias spp. e.g. E.insulana (Egyptian bollworm), Pectinophora spp. e.g. Pectinophora gossypiella (pink bollworm), Ostrinia spp. such as O.nubilalis (European cornborer), Trichoplusia ni (cabbage looper), Pieris spp. (cabbage worms), Laphygma spp. (army worms), Agrotis and Amathes spp. (cutworms), Wiseana spp. (porina moth), Chilo spp. (rice stem borer), Tryporyza spp. and Diatraea spp. (sugar cane borers and rice borers), Sparganothis pilleriana (grape berry moth), Cydia pomonella (codling moth), Archips spp. (fruit tree tortrix moths), Plutella xylostella (diamond back moth); against adult and larvae of Coleoptera (beetles) e.g. Hypothenemus hampei (coffee berry borer), Hylesinus spp. (bark beetles), Anthonomus grandis (cotton boll weevil), Acalymma spp. (cucumber beetles), Lema spp., Psylliodes spp., Leptinotarsa decemlineata (Colorado potato beetle), Diabrotica spp. (corn rootworms), Gonocephalum spp. (false wire worms), Agriotes spp. (wireworms), Dermolepida and Heteronychus spp. (white grubs), Phaedon cochleariae (mustard beetle), Lissorhoptrus oryzophilus (rice water weevil), Meligethes spp. (pollen beetles), Ceutorhynchus spp., Rhynchophorus and Cosmopolites spp. (root weevils); against Hemiptera e.g. Psylla spp., Bemisia spp., Trialeurodes spp., Aphis spp., Myzus spp., . Megoura viciae, Phylloxera spp., Adelges spp., Phorodon humuli (hop damson aphid), Aeneolamia spp., Nephotettix spp. (rice leaf hoppers), Empoasca spp., Nilaparvata spp., Perkinsiella spp., Pyrilla spp., Aonidiella spp. (red scales), Coccus spp., Pseudococcus spp., Helopeltis spp. (mosquito bugs), Lygus spp., Dysdercus spp., Oxycarenus spp., Nezara spp.; Hymenoptera e.g. Athalia spp. and Cephus spp. (saw flies), Atta spp. (leaf cutting ants); Diptera e.g. Hylemyia spp. (root flies), Atherigona spp. and Chlorops spp. (shoot flies), Phytomyza spp. (leaf miners), Ceratitis spp. (fruit flies); Thysanoptera such as Thrips tabaci; Orthoptera such as Locusta and Schistocerca spp. (locusts) and crickets e.g. Gryllus spp. and Acheta spp.; Collembola e.g. Sminthurus spp. and Onychiurus spp. (springtails), Isoptera e.g. Odontotermes spp. (termites), Dermaptera e.g. Forficula spp. (earwigs) and also other arthropods of agricultural significance such as Acari (mites) e.g. Tetranychus spp., Panonychus spp. and Bryobia spp. (spider mites), Eriophyes spp. (gall mites), Polyphagotarsonemus spp.; Blaniulus spp. (millipedes), Scutigerella spp. (symphilids), Oniscus spp. (woodlice) and Triops spp. (crustacea); nematodes which attack plants and trees of importance to agriculture, forestry and horticulture either directly or by spreading bacterial, viral, mycoplasma or fungal diseases of the plants, root-knot nematodes such as Meloidogyne spp. (e.g. M. incognita); cyst nematodes such as Globodera spp. (e.g. G. rostochiensis); Heterodera spp. (e.g. H. avenae); Radopholus spp. (e.g. R. similis); lesion nematodes such as Pratylenchus spp. (e.g. P. pratensis); Belonolaimus spp. (e.g. B. gracilis); Tylenchulus spp. (e.g. T. semipenetrans); Rotylenchulus spp. (e.g. R. reniformis); Rotylenchus spp. (e.g. R. robustus); Helicotylenchus spp. (e.g. H. multicinctus); Hemicycliophora spp. (e.g. H. gracilis); Criconemoides spp. (e.g. C. similis); Trichodorus spp. (e.g. T. primitivus); dagger nematodes such as Xiphinema spp. (e.g. X. diversicaudatum), Longidorus spp. (e.g. L. elongatus); Hoplolaimus spp. (e.g. H. coronatus); Aphelenchoides spp. (e.g. A. ritzema-bosi, A. besseyi); stem and bulb eelworms such as Ditylenchus spp. (e.g. D. dipsaci).

The invention also provides a method for the control of arthropod or nematode pests of plants which comprises the application to the plants or to the medium in which they grow of an effective amount of a compound of general formula (I).

For the control of arthropods and nematodes, the active compound is generally applied to the locus in which arthropod or nematode infestation is to be controlled at a rate of about 0.1kg to about 25kg of active compound per hectare of locus treated. Under ideal conditions, depending on the pest to be controlled, the lower rate may offer adequate protection. On the other hand, adverse weather conditions, resistance of the pest and other factors may require that the active ingredient be used in higher proportions. In foliar application, a rate of 1g to 1000g/ha may be used.

When the pest is soil-borne, the formulation containing the active compound is distributed evenly over the area to be treated in any convenient manner. Application may be made, if desired, to the field or crop-growing area generally or in close proximity to the seed or plant to be protected from attack. The active component can be washed into the soil by spraying with water over the area or can be left to the natural action of rainfall.

During or after application, the formulation can, if desired, be distributed mechanically in the soil, for example by ploughing or disking. Application can be prior to planting, at planting, after planting but before sprouting has taken place or after sprouting.

The compounds of general formula (I) may be applied in solid or liquid compositions to the soil principally to control those nematodes dwelling therein but also to the foliage principally to control those nematodes attacking the aerial parts of the plants (e.g. Aphelenchoides spp. and Ditylenchus spp. listed above).

The compounds of general formula (I) are of value in controlling pests which feed on parts of the plant remote from the point of application, e.g. leaf feeding insects are killed by the subject compounds applied to roots.

In addition the compounds may reduce attacks on the plant by means of antifeeding or repellent effects.

The compounds of general formula (I) are of particular value in the protection of field, forage, plantation, glasshouse, orchard and vineyard crops, of ornamentals and of plantation and forest trees, for example,

cereals (such as maize, wheat, rice, sorghum), cotton, tobacco, vegetables and salads (such as beans, cole crops, curcurbits, lettuce, onions, tomatoes and peppers), field crops (such as potato, sugar beet, ground nuts, soyabean, oil seed rape), sugar cane, grassland and forage (such as maize, sorghum, lucerne), plantations (such as of tea, coffee, cocoa, banana, oil palm, coconut, rubber, spices), orchards and groves (such as of stone and pip fruit, citrus, kiwifruit, avocado, mango, olives and walnuts), vineyards, ornamental plants, flowers and shrubs under glass and in gardens and parks, forest trees (both deciduous and evergreen) in forests, plantations and nurseries.

They are also valuable in the protection of timber (standing, felled, converted, stored or structural) from attack by sawflies (e.g. Urocerus) or beetles (e.g. scolytids, platypodids, lyctids, bostrychids, cerambycids, anobiids) or termites, for example Reticulitermes spp., Heterotermes spp., Coptotermes spp..

They have applications in the protection of stored products such as grains, fruits, nuts, spices and tobacco, whether whole, milled or compounded into products, from moth, beetle and mite attack. Also protected are stored animal products such as skins, hair, wool and feathers in natural or converted form (e.g. as carpets or textiles) from moth and beetle attack; also stored meat and fish from beetle, mite and fly attack.

The compounds of general formula (I) are of particular value in the control of arthropods, helminths or protozoa which are injurious to, or spread or act as vectors of diseases in man and domestic animals, for example those hereinbefore mentioned, and more especially in the control of ticks, mites, lice, fleas, midges and biting, nuisance and myiasis flies. The compounds of general formula (I) are particularly useful in controlling arthropods, helminths or protozoa which are present inside domestic host animals or which feed in or on the skin or suck the blood of the animal, for which purpose they may be administered orally, parenterally, percutaneously or topically.

Coccidiosis, a disease caused by infections by protozoan parasites of the genus Eimeria, is an important potential cause of economic loss in domestic animals and birds, particularly those raised or kept under intensive conditions. For example, cattle, sheep, pigs and rabbits may be affected, but the disease is especially important in poultry, in particular chickens.

The poultry disease is generally spread by the birds picking up the infectious organism in droppings on contaminated litter or ground or by way of food or drinking water. The disease is manifested by hemorrhage, accumulation of blood in the ceca, passage of blood to the droppings, weakness and digestive disturbances. The disease often terminates in the death of the animal but the fowl which survive severe infections have had their market value substantially reduced as a result of the infection.

Administration of a small amount of a compound of general formula (I) preferably by combination with poultry feed is effective in preventing or greatly reducing the incidence of coccidiosis. The compounds are effective against both the cecal form (caused by E. tenella) and the intestinal forms (principally caused by E. acervulina, E. brunetti, E. maxima and E. necatrix).

The compounds of general formula (I) also exert an inhibitory effect on the oocysts by greatly reducing the number and or the sporulation of those produced.

The compositions hereinafter described for topical application to man and animals and in the protection of stored products, household goods, property and areas of the general environment may, in general, alternatively be employed for application to growing crops and crop growing loci and as a seed dressing.

Suitable means of applying the compounds of general formula (I) include:

to persons or animals infested by or exposed to infestation by arthropods, helminths or protozoa, by parenteral, oral or topical application of compositions in which the active ingredient exhibits an immediate and/or prolonged action over a period of time against the arthropods, helminths or protozoa, for example by incorporation in feed or suitable orally-ingestible pharmaceutical formulations, edible baits, salt licks, dietary supplements, pour-on formulations, sprays, baths, dips, showers, jets, dusts, greases, shampoos, creams, wax-smears and livestock self-treatment systems;

to the environment in general or to specific locations where pests may lurk, including stored products, timber, household goods, and domestic and industrial premises, as sprays, fogs, dusts, smokes, wax-smears, lacquers, granules and baits, and in tricklefeeds to waterways, wells, reservoirs and other running or standing water;

to domestic animals in feed to control fly larvae feeding in their faeces;

to growing crops as foliar sprays, dusts, granules, fogs and foams; also as suspensions of finely divided and encapsulated compounds of general formula (I); as soil and root treatments by liquid drenches, dusts, granules, smokes and foams; and as seed dressings by liquid slurries and dusts.

The compounds of general formula (I) may be applied to control arthropods, helminths or protozoa in compositions of any type known to the art suitable for internal or external administration to vertebrates or application for the control of arthropods in any premises or indoor or outdoor area, containing as active ingredient at least one compound of general formula (I) in association with one or more compatible diluents or adjuvants appropriate for the intended use. All such compositions may be prepared in any manner known to the art.

Compositions suitable for administration to vertebrates or man include preparations suitable for oral, parenteral, percutaneous, e.g. pour-on, or topical administration.

Compositions for oral administration comprise one or more of the compounds of general formula (I) in association with pharmaceutically acceptable carriers or coatings and include, for example, tablets, pills,

capsules, pastes, gels, drenches, medicated feeds, medicated drinking water, medicated dietary supplements, slow-release boluses or other slow-release devices intended to be retained within the gastro-intestinal tract. Any of these may incorporate active ingredient contained within microcapsules or coated with acid-labile or alkali-labile or other pharmaceutically acceptable enteric coatings. Feed premixes and concentrates containing compounds of the present invention for use in preparation of medicated diets, drinking water or other materials for consumption by animals may also be used.

Compositions for parenteral administration include solutions, emulsions or suspensions in any suitable pharmaceutically acceptable vehicle and solid or semisolid subcutaneous implants or pellets designed to release active ingredient over a protracted period and may be prepared and made sterile in any appropriate manner known to the art.

Compositions for percutaneous and topical administration include sprays, dusts, baths, dips, showers, jets, greases, shampoos, creams, wax-smears, or pour-on preparations and devices (e.g. ear tags) attached externally to animals in such a way as to provide local or systemic arthropod control.

Solid or liquid baits suitable for controlling arthropods comprise one or more compounds of general formula (I) and a carrier or diluent which may include a food substance or some other substance to induce consumption by the arthropod.

Liquid compositions include water miscible concentrates, emulsifiable concentrates, flowable suspensions, wettable or soluble powders containing one or more compounds of general formula (I) which may be used to treat substrates or sites infested or liable to infestation by arthropods including premises, outdoor or indoor storage or processing areas, containers or equipment and standing or running water.

Solid homogenous or heterogenous compositions containing one or more compounds of general formula (I), for example granules, pellets, briquettes or capsules, may be used to treat standing or running water over a period of time. A similar effect may be achieved using trickle or intermittent feeds of water dispersible concentrates as described herein.

Compositions in the form of aerosols and aqueous or non-aqueous solutions or dispersions suitable for spraying, fogging and low- or ultra-low volume spraying may also be used.

Suitable solid diluents which may be used in the preparation of compositions suitable for applying the compounds of general formula (I) include aluminium silicate, kieselguhr, corn husks, tricalcium phosphate, powdered cork, absorbent carbon black, magnesium silicate, a clay such as kaolin, bentonite or attapulgite, and water soluble polymers and such solid compositions may, if desired, contain one or more compatible wetting, dispersing, emulsifying or colouring agents which, when solid, may also serve as diluent.

Such solid compositions, which may take the form of dusts, granules or wettable powders, are generally prepared by impregnating the solid diluents with solutions of the compound of general formula (I) in volatile solvents, evaporating the solvents and, if necessary, grinding the products so as to obtain powders and, if desired, granulating or compacting the products so as to obtain granules, pellets or briquettes or by encapsulating finely divided active ingredient in natural or synthetic polymers, e.g. gelatin, synthetic resins and polyamides.

The wetting, dispersing and emulsifying agents which may be present, particularly in wettable powders, may be of the ionic or non-ionic types, for example sulphoricinoleates, quaternary ammonium derivatives or products based upon condensates of ethylene oxide with nonyl- and octyl-phenol, or carboxylic acid esters of anhydro-sorbitols which have been rendered soluble by etherification of the free hydroxy groups by condensation with ethylene oxide, or mixtures of these types of agents. Wettable powders may be treated with water immediately before use to give suspensions ready for application.

Liquid compositions for the application of the compounds of general formula (I) may take the form of solutions, suspensions and emulsions of the compounds of general formula (I) optionally encapsulated in natural or synthetic polymers, and may, if desired, incorporate wetting, dispersing or emulsifying agents. These emulsions, suspensions and solutions may be prepared using aqueous, organic or aqueous-organic diluents, for example acetophenone, isophorone, toluene, xylene, mineral, animal or vegetable oils, and water soluble polymers (and mixtures of these diluents), which may contain wetting, dispersing or emulsifying agents of the ionic or non-ionic types or mixtures thereof, for example those of the types described above. When desired, the emulsions containing the compounds of general formula (I) may be used in the form of self-emulsifying concentrates containing the active substance dissolved in the emulsifying agents or in solvents containing emulsifying agents compatible with the active substance, the simple addition of water to such concentrates producing compositions ready for use.

Compositions containing compounds of general formula (I) which may be applied to control arthropod, plant nematode, helminth or protozoan pests, may also contain synergists (e.g. piperonyl butoxide or sesamex), stabilizing substances, other insecticides, acaricides, plant nematocides, anthelmintics or anticoccidials, fungicides (agricultural or veterinary as apropriate e.g. benomyl, iprodione), bactericides, arthropod or vertebrate attractants or repellents or pheromones, reodorants, flavouring agents, dyes and auxiliary therapeutic agents, e.g. trace elements. These may be designed to improve potency, persistence, safety, uptake where desired, spectrum of pests controlled or to enable the composition to perform other useful functions in the same animal or area treated.

Examples of other pesticidally-active compounds which may be included in, or used in conjunction with, the compositions of the present invention are:- acephate, chlorpyrifos, demeton-S-methyl, disulfoton, ethoprofos, fenitrothion, malathion, monocrotophos, parathion, phosalone, pirimiphos-methyl, triazophos, cyfluthrin,

cypermethrin, deltamethrin, fenpropathrin, fenvalerate, permethrin, aldicarb, carbosulfan, methomyl, oxamyl, pirimicarb, bendiocarb, teflubenzuron, dicofol, endosulfan, lindane, benzoximate, cartap, cyhexatin, tetradifon, avermectins, ivermectin, milbemycins, thiophanate, trichlorfon, dichlorvos, diaveridine and dimetridazole.

The compositions for application to control arthropod, plant nematode, helminth or protozoan pests usually contain from 0.00001% to 95%, more particularly from 0.0005% to 50%, by weight of one or more compounds of general formula (I) or of total active ingredients (that is to say the compound(s) of general formula (I) together with other substances toxic to arthropods and plant nematodes, anthelmintics, anticoccidials, synergists, trace elements or stabilisers). The actual compositions employed and their rate of application will be selected to achieve the desired effect(s) by the farmer, livestock producer, medical or veterinary practitioner, pest control operator or other person skilled in the art. Solid and liquid compositions for application topically to animals, timber, stored products or household goods usually contain from 0.00005% to 90%, more particularly from 0.001% to 10%, by weight of one or more compounds of general formula (I). For administration to animals orally or parenterally, including percutaneously solid and liquid compositions normally contain from 0.1% to 90% by weight of one or more compound of general formula (I). Medicated feedstuffs normally contain from 0.001% to 3% by weight of one or more compounds of general formula (I). Concentrates and supplements for mixing with feedstuffs normally contain from 5% to 90%, and preferably from 5% to 50%, by weight of one or more compounds of general formula (I). Mineral salt licks normally contain from 0.1% to 10% by weight of one or more compounds of general formula (I).

Dusts and liquid compositions for application to livestock, persons, goods, premises or outdoor areas may contain 0.0001% to 15%, and more especially 0.005% to 2.0%, by weight of one or more compounds of general formula (I). Suitable concentrations in treated waters are between 0.0001 ppm and 20 ppm, and more especially 0.001 ppm to 5.0 ppm. of one or more compounds of general formula (I) and may also be used therapeutically in fish farming with appropriate exposure times. Edible baits may contain from 0.01% to 5% and preferably 0.01% to 1.0%, by weight of one or more compounds of general formula (I).

When administered to vertebrates parenterally, orally or by percutaneous or other means, the dosage of compounds of general formula (I) will depend upon the species, age and health of the vertebrate and upon the nature and degree of its actual or potential infestation by arthropod, helminth or protozoan pest. A single dose of 0.1 to 100 mg, preferably 2.0 to 20.0 mg, per kg body weight of the animal or doses of 0.01 to 20.0 mg, preferably 0.1 to 5.0 mg, per kg body weight of the animal per day for sustained medication are generally suitable by oral or parenteral administration. By use of sustained release formulations or devices, the daily doses required over a period of months may be combined and administered to animals on a single occasion.

In experiments on activity against arthropods carried out on representative compounds, the following results (wherein ppm indicates the concentration of the compound in parts per million of the test solution applied) have been obtained:-

Test 1

One or more of the dilutions of the compounds to be tested were made in 50% aqueous acetone. Test species : Spodoptera littoralis (Egyptian cotton worm).

French bean leaf discs were set in agar in petri dishes and infected with 5 2nd instar larvae. Four replicate dishes were assigned to each treatment and were sprayed under a Potter Tower with the appropriate test dilution. After 2 days, live larvae were transferred to similar dishes containing untreated leaves set in agar. 2 or 3 days later, the dishes were removed from the constant temperature (25°C) room in which they had been held and the mean percentage mortalities of larvae were determined. These data were corrected against the mortalities in dishes treated with 50% aqueous acetone alone which served as controls.

According to the above method an application of 500 ppm of the following compounds was effective against the larvae of Spodoptera littoralis, producing at least 70% mortality:

Compounds 1, 2, 3, 4, 5, 6.

The following Composition Examples illustrate compositions for use against arthropod, plant nematode, helminth or protozoan pests which comprise, as active ingredient, compounds of general formula (I). The compositions described in Composition Examples 1 to 6 can each be diluted in water to give a sprayable composition at concentrations suitable for use in the field.

COMPOSITION EXAMPLE 1

A water soluble concentrate was prepared from

5-Acetamido-3-cyano-l-(2,6-dichloro-4-trifluoromethylphenyl)-4-nitropyrazole 7% w/v
Ethylan BCP 10% w/v
and N-methylpyrrolidone to100% by volume

by dissolving the Ethylan BCP in a portion of N-methylpyrrolidone, and then adding the active ingredient with heating and stirring until dissolved. The resulting solution was made up to volume by adding the remainder of the solvent.

COMPOSITION EXAMPLE 2

An emulsifiable concentrate was prepared from

5-Acetamido-3-cyano-1-(2,6-dichloro-4-trifluoromethylphenyl)-4-nitropyrazole 7% w/v
Soprophor BSU 4% w/v

Arylan CA 4% w/v
N-methylpyrrolidone 50% w/v
and Solvesso 150 to 100% by volume
by dissolving Soprophor BSU, Arylan CA and the active ingredient in N-methylpyrrolidone, and then adding Solvesso 150 to volume.

COMPOSITION EXAMPLE 3

A wettable powder was prepared from
5-Acetamido-3-cyano-1-(2,6-dichloro-4-trifluoromethylphenyl)-4-nitropyrazole 40% w/w
Arylan S 2% w/w
Darvan No. 2 5% w/w
and Celite PF to 100% by weight
by mixing the ingredients, and grinding the mixture in a hammer-mill to a particle size less than 50 microns.

COMPOSITION EXAMPLE 4

An aqueous flowable formulation was prepared from
5-Acetamido-3-cyano-l-(2,6-dichloro-4-trifluoromethylphenyl)-4-nitropyrazole 30% w/v
Ethylan BCP 1% w/v
Sopropon T36 0.2%w/v
Ethylene glycol 5% w/v
Rhodigel 23 0.15% w/v
and Water to 100% by volume
by intimately mixing the ingredients and grinding in a bead mill until the median particle size was less than 3 microns.

COMPOSITION EXAMPLE 5

An emulsifiable suspension concentrate was prepared from
5-Acetamido-3-cyano-l-(2,6-dichloro-4-trifluoromethylphenyl)-4-nitropyrazole 30% w/v
Ethylan BCP 10% w/v
Bentone 38 0.5% w/v
and Solvesso 150 to 100% by volume
by intimately mixing the ingredients and grinding in a bead mill until the median particle size was less than 3 microns.

COMPOSITION EXAMPLE 6

Water dispersible granules were prepared from
5-Acetamido-3-cyano-1-(2,6-dichloro-4-trifluoromethylphenyl)-4-nitropyrazole 30% w/w
Darvan No. 2 15% w/w
Arylan S 8% w/w
and Celite PF to 100% by weight
by mixing the ingredients, micronising in a fluid-energy mill, and then granulating in a rotating pelletiser by spraying on sufficient water (up to 10% w/w). The resulting granules were dried in a fluid-bed drier to remove excess water.

Descriptions of commercial ingredients used in the foregoing Composition Examples:-

Ethylan BCP nonylphenol ethylene oxide condensate
Soprophor BSU condensate of tristyrylphenol and ethylene oxide
Arylan CA 70% w/v solution of calcium dodecylbenzenesulphonate
Solvesso 150 light $C_{10}$-aromatic solvent
Arylan S sodium dodecylbenzenesulphonate
Darvan sodium lignosulphonate
Celite PF synthetic magnesium silicate carrier
Sopropon T36 sodium salt of polycarboxylic acid
Rhodigel 23 polysaccharide xanthan gum
Bentone 38 organic derivative of magnesium montmorillonite

COMPOSITION EXAMPLE 7

A dusting powder may be prepared by intimately mixing:-
5-Acetamido-3-cyano-1-(2,6-dichloro-4-trifluoromethylphenyl)-4-nitropyrazole 1 to 10% w/w (weight/weight)
Talc superfine to 100% by weight.

This powder may be applied to a locus of arthropod infestation, for example refuse tips or dumps, stored products or household goods or animals infested by, or at risk of infestation by, arthropods to control the arthropods by oral ingestion. Suitable means for distributing the dusting powder to the locus of arthropod infestation include mechanical blowers, handshakers and livestock self treatment devices.

COMPOSITION EXAMPLE 8

An edible bait may be prepared by intimately mixing:-

5-Acetamido-3-cyano-1-(2,6-dichloro-4-trifluoromethylphenyl)-4-nitropyrazole 0.1 to 1.0% w/w

Wheat flour 80% w/w

Molasses to 100% w/w

This edible bait may be distributed at a locus, for example domestic and industrial premises, e.g. kitchens, hospitals or stores, or outdoor areas, infested by arthropods, for example ants, locusts, cockroaches and flies, to control the arthropods by oral ingestion.

COMPOSITION EXAMPLE 9

A solution may be prepared containing:-

5-Acetamido-3-cyano-l-(2,6-dichloro-4-trifluoromethylphenyl)-4-nitropyrazole 15% w/v (weight/volume)

Dimethylsulphoxide to 100% by volume

by dissolving the pyrazole derivative in a portion of the dimethyl- sulphoxide and then adding more dimethylsulphoxide to the desired volume.

This solution may be applied to domestic animals infested by arthropods, percutaneously as a pour-on application or, after sterilisation by filtration through a polytetrafluoroethylene membrane (0.22 micrometre pore size), by parenteral injection, at a rate of application of from 1.2 to 12 ml of solution per 100 kg of animal body weight.

COMPOSITION EXAMPLE 10

A wettable powder may be formed from:-

5-Acetamido-3-cyano-l-(2,6-dichloro-4-trifluoromethylphenyl)-4-nitropyrazole 50% w/w

Ethylan BCP (a nonylphenol/ethylene oxide condensate containing 9 moles of ethylene oxide per mol of phenol) 5% w/w

Aerosil (silicon dioxide of microfine-particle size) 5% w/w

Celite PF synthetic magnesium silicate carrier) 40% w/w

by adsorbing the Ethylan BCP onto the Aerosil, mixing with the other ingredients and grinding the mixture in a hammer-mill to give a wettable powder, which may be diluted with water to a concentration of from 0.001% to 2% w/v of the pyrazole compound and applied to a locus of infestation by arthropods, for example dipterous larvae, or plant nematodes by spraying, or to domestic animals infested by, or at risk of infestation by, arthropods, helminths or protozoa, by spraying or dipping, or by oral administration as drinking water, to control the arthropods, helminths or protozoa.

COMPOSITION EXAMPLE 11

A slow release bolus may be formed from granules containing a density agent, binder, slow-release agent and 5-acetamido-3-cyano-1-(2,6-dichloro-4-trifluoromethylphenyl)-4-nitropyrazole compound at varying percentage compositions. By compressing the mixture, a bolus with a specific gravity of 2 or more can be formed and may be administered orally to ruminant domestic animals for retention within the reticulo-rumen to give a continual slow release of pyrazole compound over an extended period of time to control infestation of the ruminant domestic animals by arthropods, helminths or protozoa.

COMPOSITION EXAMPLE 12

A slow release composition may be prepared from:-

5-Acetamido-3-cyano-1-(2,6-dichloro-4-trifluoromethylphenyl)-4-nitropyrazole 0.5 to 25% w/w

polyvinylchloride base to 100% w/w

by blending the polyvinylchloride base with the pyrazole compound and a suitable plasticiser, e.g. dioctyl phthalate, and melt-extruding or hot-moulding the homogenous composition into suitable shapes, e.g. granules, pellets, brickettes or strips, suitable, for example, for addition to standing water or, in the case of strips, fabrication into collars or ear-tags for attachment to domestic animals, to control insect pests by slow release of the pyrazole compound.

Similar compositions may be prepared by replacing the 5-acetamido-3-cyano-l-(2,6-dichloro-4-trifluoromethylphenyl)-4-nitropyrazole in the Composition Examples by the appropriate quantity of any other compound of general formula (I).

The compounds of general formula I can be prepared by the application or adaption of known methods (i.e. methods heretofore used or described in the chemical literature), generally heterocycle formation followed where necessary by changing substituents with protection/deprotection of other substituents if necessary (e.g. acylation of amino groups), for example as hereinafter described.

In the following description when symbols appearing in formulae are not specifically defined it is to be understood that they are "as hereinbefore defined" in accordance with the first definition of each symbol in this specification. Within the process definitions, unless otherwise stated, amino refers to the unsubstituted amino group.

It will be appreciated that in the preparation of compounds of general formula I the following processes or

adaptations thereof may be performed in an appropriate combination to achieve the compound sought (for example hydrolysis of acylated amino to unsubstituted amino): compounds of general formula I may be converted by known methods into other compounds of general formula I.

According to process version 'a', which is a further feature of the present invention, compounds of general formula I, wherein $R^5$ represents an amino group $-NHR^{7'}$, wherein $R^{7'}$ represents the hydrogen atom or a straight- or branched-chain alkyl group containing from 1 to 6 carbon atoms may be prepared from compounds of general formula I, wherein $R^5$ represents an amino group $-NR^{6'}R^{7''}$, wherein $R^{6'}$ represents the formyl group or a straight- or branched-chain alkanoyl group containing from 2 to 7 carbon atoms which may be unsubstituted or substituted by one or more halogen atoms, or a straight-or branched-chain alkoxycarbonyl group containing from 2 to 7 carbon atoms, which may be unsubstituted or substituted by one or more halogen atoms, and $R^{7''}$ is as hereinbefore defined for $R^7$ except that $R^{6'}$ and $R^{7''}$ together do not form a 5 or 6 membered cyclic imide with the nitrogen atom to which they are attached, by hydrolysis. The hydrolysis is generally effected with an acid such as hydrochloric acid or hydrobromic acid in a solvent such as acetic acid or dioxan, or with an alkali metal, e.g. sodium or potassium, hydroxide in water or an inert organic or aqueous-organic solvent, e.g. a lower alkanol such as methanol or a mixture of water and a lower alkanol, at a temperature from laboratory temperature to the boiling point of the reaction mixture.

According to process version 'b', which is a further feature of the present invention, compounds of general formula I, wherein $R^5$ is as hereinbefore defined but does not represent an unsubstituted amino or alkylamino group, may be prepared from the corresponding compounds within general formula II, wherein $R^1$, $R^2$, $R^3$ and $R^4$ are as hereinbefore defined and $R^5$ is replaced by $R^{5'}$ which is as hereinbefore defined for $R^5$ but does not represent an unsubstituted amino or alkylamino group (such compounds are referred to as of general formula IIA), by treatment with a nitrating agent, preferably nitric acid, optionally in the presence of sulphuric acid, or nitric acid in a solvent such as acetic acid or acetic anhydride, at a temperature from 0°C to 100°C.

The preparation of derivatives of the 5-amino group form a further feature of the present invention and are collectively referred to as process 'c'. Compounds of general formula I which conform to general formula IB, wherein $R^6$ represents an $R^9C(=0)$- group, wherein $R^9$ represents a straight- or branched-chain alkyl or alkoxy group containing from 1 to 6 carbon atoms which is unsubstituted or substituted by one or more halogen atoms, and $R^7$ represents a hydrogen atom or an $R^9C(=0)$- group which is identical to the group $R^9C(=0)$- represented by $R^6$, or $-NR^6R^7$ represents a cyclic imide as hereinbefore defined, may be prepared by the reaction of a compound of general formula I wherein $R^5$ represents the unsubstituted amino group, or an alkali metal salt thereof, with a compound of the general formula:-

$R^9COX^1$ III

wherein $X^1$ represents a chlorine or bromine atom, or with a compound of the general formula:-

$(R^9CO)_20$ IV

or with a dicarboxylic acid derivatives. The reaction may be conducted in the absence or presence of an inert organic solvent, for example acetonitrile, tetrahydrofuran, a ketone, e.g. acetone, an aromatic hydrocarbon, e.g. benzene or toluene, chloroform, dichloromethane or dimethylformamide, and optionally in the presence of an acid-binding agent, for example pyridine, triethylamine or an alkali metal, e.g. sodium or potassium, carbonate or bicarbonate, at a temperature from 0°C to the reflux temperature of the reaction medium, to give a compound of general formula IB, wherein $R^6$ represents an $R^9C(=0)$- group wherein $R^9$ is as hereinbefore defined and $R^7$ represents a hydrogen atom or an $R^9C(=0)$- group, depending upon the reaction conditions chosen and/or the use of an excess of the compound of general formula III or IV, or $-NR^6R^7$ represents a cyclic imide as hereinbefore defined.

Compounds of general formula IB, wherein $R^6$ represents a formyl group and $R^7$ represents a hydrogen atom or a formyl group, may be prepared by the reaction of a compound of general formula I, wherein $R^5$ represents the unsubstituted amino group with formylacetic anhydride. Formylacetic anhydride may be prepared from formic acid and acetic anhydride and the reaction with the compound of general formula I may be conducted in the absence or presence of an inert organic solvent, for example a ketone, e.g. acetone, or an aromatic hydrocarbon, e.g. benzene or toluene, and optionally in the presence of an acid-binding agent, for example pyridine, triethylamine or an alkali metal, e.g. sodium or potassium, carbonate or bicarbonate, at a temperature from 0°C to the reflux temperature of the reaction mixture, to give a compound of general formula IB wherein $R^6$ represents a formyl group and $R^7$ represents a hydrogen atom or a formyl group, depending upon the reaction conditions chosen and/or the use of an excess of formyl-acetic anhydride.

Compounds of general formula IB wherein $R^6$ represents a formyl group or a group $R^9C(=0)$- and $R^7$ represents a hydrogen atom may be prepared by the selective removal by hydrolysis of an $R^9C(=0)$- group or a formyl group from a compound of general formula IB wherein $R^6$ and $R^7$ both represent a $R^9C(=0)$ group or a formyl group. Hydrolysis is effected under mild conditions, for example by treatment with an aqueous-ethanolic solution or suspension of an alkali metal, e.g. sodium or potassium, bicarbonate, or with aqueous ammonia.

Compounds of general formula IB, wherein $R^6$ represents a straight- or branched-chain alkoxycarbonyl group containing from 2 to 7 carbon atoms which is unsubstituted or substituted by one or more halogen atoms, and $R^7$ represents a hydrogen atom may be prepared by the reaction of a compound of the general formula V wherein $R^{10}$ represents an alkoxycarbonyl group $R^{11}C(=0)$, wherein $R^{11}$ represents a straight- or branched-chain alkoxy group containing from 1 to 6 carbon atoms (which is unsubstituted or substituted by one or more halogen atoms) or a phenoxy group, with a compound of the general formula:-

$R^{11}H$ VI

to replace a first group represented by the symbol $R^{10}$ by a hydrogen atom, and to replace the second group represented by the symbol $R^{10}$ by an alkoxycarbonyl group when $R^{10}$ represents a phenoxycarbonyl group, or, if desired, to replace the second group represented by the symbol $R^{10}$ by another alkoxycarbonyl group when $R^{10}$ in formula V represents an alkoxycarbonyl group. As will be apparent to those skilled in the art, the desired compound of general formula IB is obtained by selection of the appropriate compounds of general formulae V and VI. The reaction may be effected in water or an inert aqueous- organic or organic solvent, for example an alkanol containing 1 to 4 carbon atoms, e.g. ethanol, or an aromatic hydrocarbon, e.g. benzene or toluene, which is preferably an excess of the compound of general formula VI, at a temperature from ambient temperature to the reflux temperature of the reaction mixture and, if necessary, at elevated pressure, and optionally in the presence of a base, for example an alkali metal alkoxide, e.g. of the compound of general formula VI.

Compounds of general formula IB wherein $R^6$ and $R^7$, which may be the same or different, each represents a formyl group or a $R^9C(=O)$- group, may be prepared by the reaction of an alkali metal, e.g. sodium or potassium, derivative of a compound of general formula IB wherein $R^6$ represents a group $R^9C(=O)$- as hereinbefore defined, or a formyl group, and $R^7$ represents a hydrogen atom with formylacetic anhydride or a compound of general formula III. Reaction may be effected in an inert aprotic solvent, e.g. dimethylformamide, at a temperature from laboratory temperature to the reflux temperature of the reaction mixture.

Alkali metal derivatives of compounds of general formula I (wherein $R^5$ represents the unsubstituted amino group) or IB wherein $R^7$ represents a hydrogen atom may be prepared in situ by reaction with an alkali metal, e.g. sodium or potassium, hydride, in an inert aprotic solvent, e.g. dimethylformamide, at a temperature from laboratory temperature to the reflux temperature of the reaction mixture.

Compounds of general formula V wherein $R^{10}$ represents a group $R^{11}C(=O)$-, may be prepared as hereinbefore described. Compounds of general formula V wherein $R^{10}$ represents a phenoxycarbonyl group may be prepared by the reaction of a compound of general formula I (wherein $R^5$ represents the unsubstituted amino group), with phenyl chloroformate using the reaction conditions hereinbefore described for the reaction of a compound of general formula I with a compound of formula III.

Compounds of general formula IB wherein $R^6$ represents a group $R^{12}$ which represents a straight- or branched-chain alkyl group containing from 1 to 6 carbon atoms and $R^7$ represents a hydrogen atom may be prepared by the removal of the group $R^9C(=O)$- of a compound of the general formula IB, wherein $R^6$ represents a group $R^{12}$ and $R^7$ represents a group $R^9C(=O)$-. Removal of the group $R^9C(=O)$- may be effected by selective hydrolysis under mild conditions, for example by treatment with an alkali metal, e.g. sodium or potassium, hydroxide in water or an inert organic or aqueous- organic solvent, for example a lower alkanol, e.g. methanol, or a mixture of water and lower alkanol, at a temperature from laboratory temperature up to the reflux temperature of the reaction mixture.

Compounds of general formula IB, wherein $R^6$ represents a group $R^{12}$ and $R^7$ represents a group $R^9C(=O)$-, may be prepared by reaction of a compound of general formula IB, wherein $R^6$ represents a hydrogen atom, or an alkali metal, e.g. sodium or potassium, derivative thereof, with a compound of the general formula:-

$R^{12}X$ VII

wherein X represents a chlorine, bromine or iodine atom. Reaction may be effected in an inert organic solvent, e.g. dichloromethane, tetrahydrofuran, or dimethylformamide, at a temperature from ambient up to the boiling point of the reaction mixture and, when a compound of general formula IB is used, in the presence of a base, e.g. Triton B; or by reaction of a compound of general formula IB wherein $R^6$ represents the hydrogen atom and $R^7$ represents a group $R^{12}$ with a compound of general formula III or IV.

Compounds of general formula I, wherein $R^5$ represents an N-alkyl-N-formylamino group as hereinbefore described may be prepared in a similar manner to the process above using, where appropriate, formylacetic anhydride instead of a compound of general formula III or IV.

Compounds of general formula IB, wherein one or both of $R^6$ and $R^7$ represent a straight- or branched-chain alkyl group containing from 1 to 6 carbon atoms, groups represented by $R^6$ and $R^7$ being identical, may be prepared by reaction of a compound of general formula I, wherein $R^5$ represents the unsubstituted amino group, or an alkali metal, e.g. sodium or potassium, derivative thereof, with a compound of general formula VII, in the absence or presence of an inert organic solvent, for example an aromatic hydrocarbon, e.g. benzene or toluene, chloroform, dichloromethane, tetrahydrofuran or dimethylformamide, and optionally in the presence of an acid-binding agent, for example pyridine, triethylamine or an alkali metal, e.g. sodium or potassium, bicarbonate, at a temperature from 0°C up to the boiling point of the reaction mixture.

Compounds of general formula I, wherein $R^5$ represents a straight- or branched-chain alkoxymethyleneamino group containing from 2 to 5 carbon atoms which may be unsubstituted or substituted on methylene by a straight- or branched-chain alkyl group containing from 1 to 4 carbon atoms may be prepared by the reaction of a compound of general formula I (wherein $R^5$ represents the unsubstituted amino group) with a trisalkoxyalkane in the presence of an acidic catalyst, e.g. p-toluenesulphonic acid, at a temperature from ambient to the boiling point of the reaction mixture.

Compounds of general formula I wherein $R^5$ represents $-NHCH_2R^{13}$ wherein $R^{13}$ represents the hydrogen atom or a straight- or branched-chain alkyl group containing from 1 to 4 carbon atoms may be prepared by reaction of a compound of general formula I, wherein $R^5$ represents $-N=C(OR^{14})R^{13}$ wherein $R^{14}$ represents

a straight- or branched-chain alkyl group containing from 1 to 4 carbon atoms, with a reducing agent, preferably sodium borohydride. The reaction may be effected in an inert organic solvent, ethanol or methanol being preferred, at a temperature from 0°C to the boiling point of the reaction mixture.

According to process version 'd', which is a further feature of the present invention, compounds of general formula I, wherein $R^4$ represents a fluorine, chlorine, bromine or iodine atom, may be prepared by the replacement by known methods of an amino group by a halogen atom, for example by diazotisation of a compound corresponding to general formula II wherein $R^4$ is replaced by the amino group and the hydrogen atom in the 4-position of the pyrazole ring is replaced by the nitro group, using sodium nitrite in tetrafluoroboric acid and sulphuric acid at a temperature of -10 to +10°C followed by photolysis in an excess of tetrafluoroboric acid at a temperature of -30 to +30°C to obtain a compound wherein $R^4$ represents a fluorine atom , or with an alkyl nitrite, preferably tertiary butyl nitrite, in the presence of a halogenating agent, preferably anhydrous copper chloride, bromoform or iodine respectively, at a temperature from 0°C to 100°C to obtain a compound wherein $R^4$ represents a chlorine, bromine or iodine atom.

According to process version 'e', which is a further feature of the present invention compounds of general formula I, wherein $R^5$ represents a halogen atom, may be prepared by diazotisation of the corresponding compound of general formula I wherein $R^5$ represents the amino group, in a similar manner to that hereinbefore described in process version 'd'.

Compounds of the general formula II, wherein $R^4$ represents the nitro group and $R^5$ is as hereinbefore defined but not the unsubstituted amino group may be prepared by oxidation of a compound of general formula VIII, wherein $R^{4'}$ represents the amino group, $R^{5'}$ is as hereinbefore defined for $R^5$ except that $R^{5'}$ does not represent the unsubstituted amino group, and $R^1$, $R^2$ and $R^3$ are as hereinbefore defined, with trifluoroacetic peracid (which is prepared in situ from trifluoroacetic anhydride and hydrogen peroxide (85% w/w)) in dichloromethane at a temperature between ambient and the boiling point of the solvent.

Compounds of general formula VIII, wherein $R^{4'}$ represents the amino through and $R^1$, $R^2$, $R^3$ and $R^{5'}$ are as hereinbefore defined, can be prepared by performing a Curtius rearrangement of the corresponding acid azide by heating in an inert solvent such as toluene at a temperature from 50 to 150°C to give an isocyanate which is then treated with tertiary butanol at a temperature between 50 and 150°C to give a carbamate which in turn is hydrolysed using dilute hydrochloric acid in ethanol or using iodotrimethylsilane in an inert solvent such as acetonitrile at a temperature between ambient and the boiling point of the solvent.

The intermediate acid azide may be prepared from compounds corresponding to general formula VIII, wherein $R^{4'}$ is replaced by the carboxy group (prepared by hydrolysis of the corresponding ester) with an azide transfer agent preferably diphenylphosphonylazide in a solvent such as dimethyl formamide in the presence of triethylamine at a temperature from ambient to 100°C.

The intermediate ester is prepared by treating a phenylhydrazine of general formula IX with a compound of general formula X wherein $R^{15}$ preferably represents ethyl, by treatment with dilute mineral acid e.g. sulphuric acid, followed by reaction in a lower alkanol solvent, preferably ethanol, at ambient temperature to the boiling point of the solvent.

According to process version 'f', which is a further feature of the present invention, compounds of the general formula I, wherein $R^5$ represents a hydrogen atom may be prepared by treatment of a compound of general formula I wherein $R^5$ represents the amino group, with an alkyl nitrite, preferably tertiary butyl nitrite, in an inert organic solvent, preferably tetrahydrofuran, at ambient to reflux temperature.

Intermediates of general formula XI, wherein $R^{4''}$ represents the cyano group or a chlorine or fluorine atom, may be prepared by reaction of an appropriately substituted phenylhydrazine (or acid addition salt thereof) of general formula IX with tetracyanoethylene or dichloromethylenemalononitrile or difluoromethylene-malononitrile, in an inert solvent, for example ethanol or acetic acid, at a temperature from ambient to reflux, optionally (or in the case of an acid addition salt) in the presence of a base ie. potassium carbonate or triethylamine.

Intermediates corresponding to general formula II, wherein $R^4$ represents a fluorine, chlorine, bromine or iodine atom and the hydrogen atom in the 4-position of the pyrazole ring is replaced by a group $R^8$ which represents a cyano or formyl group or the group $R^{15}OC(=O)$, wherein $R^{15}$ represents an alkyl group containing from 1 to 8 carbon atoms, preferably the ethyl group, may be prepared by diazotisation of a compound corresponding to general formula II wherein $R^4$ is replaced by the amino group and the hydrogen atom in the 4-position of the pyrazole ring is replaced by a group $R^8$, using sodium nitrite in tetrafluoroboric acid and sulphuric acid at a temperature of -10 to +10°C followed by photolysis in an excess of tetrafluoroboric acid at a temperature of -30 to +30°C to obtain a compound wherein $R^4$ represents a fluorine atom, or with an alkyl nitrite, preferably tertiary butyl nitrite, in the presence of a halogenating agent, preferably anhydrous copper chloride, bromoform or iodine respectively, at a temperature from 0°C to 100°C to obtain a compound wherein $R^4$ represents a chlorine, bromine or iodine atom.

Intermediate esters corresponding to general formula II, wherein $R^4$ represents a chlorine, bromine or iodine atom, or $R^4$ represents the fluorine atom, $R^5$ represents the amino group and the hydrogen atom in the 4-position of the pyrazole ring is replaced by a group $R^{15}OC(=O)$- wherein $R^{15}$ is as hereinbefore defined, may be prepared by diazotisation of a compound of general formula XII wherein $R^{15}$ is as hereinbefore defined in a similar manner to process version 'd' as hereinbefore described.

Intermediate diaminoesters of general formula XII wherein $R^{15}$ is as hereinbefore defined may be prepared by reaction of an appropriately substituted phenylhydrazine (or acid addition salt thereof) of general formula IX with an alkali metal salt of an alkyl dicyanoacetate, preferably potassium ethyl dicyanoacetate, in hydrochloric

acid at ambient to reflux temperature. Alkyl dicyanoacetate potassium salts may be prepared by reaction of the appropriate alkyl chloroformate with malononitrile in the presence of potassium hydroxide in tetrahydrofuran at a temperature between 0 and 100°C.

Intermediates aldehydes corresponding to general formula II, wherein $R^4$ represents the cyano group or a chlorine or fluorine atom and the hydrogen atom in the 4-position of the pyrazole ring is replaced by the formyl group, may be prepared by reduction of a compound of general formula XI, wherein $R^{4''}$ is as defined above, with Raney nickel in formic acid, at reflux or with di-isobutylaluminium hydride in an inert solvent such as tetrahydrofuran at -78 to 0°C.

Intermediates of general formula II, wherein $R^5$ represents the hydrogen atom, may be prepared by treatment of the corresponding compound of general formula II, wherein $R^5$ represents the amino group, in a similar manner to process version 'f' as hereinbefore described.

Intermediates of general formula XIII wherein $R^4$ represents a halogen atom or the nitro group may be prepared by reaction of compounds corresponding to general formula II, wherein $R^4$ represents a halogen atom or the nitro group, $R^5$ represents the amino group and the hydrogen atom in the 4-position of the pyrazole ring is replaced by a formyl or $R^{15}OC(=O)$- group, by heating at reflux in a mixture of a mineral acid, e.g. hydrochloric acid, and acetic acid.

Intermediates of general formula II, wherein $R^4$ represents bromine or iodine can be prepared by refluxing compounds of general formula II wherein $R^4$ represents chlorine in a mixture of acetic acid and either hydrobromic or hydroiodic acid respectively.

Intermediates of general formula XIII wherein $R^4$ represents the cyano group may be prepared by diazotisation of the appropriate aniline with a solution of a molar equivalent of sodium nitrite in a mineral acid, e.g. a mixture of concentrated sulphuric acid and acetic acid at a temperature from 0 to 60°C, and then reacting with the compound of general formula $NCCH_2CH(CN)COR^o$ wherein $R^o$ represents an alkoxy, preferably ethoxy, group in the presence of an inert solvent, e.g. a mixture of water and ethanol, buffered, e.g. with excess sodium acetate, and at a temperature from 0 to 50°C.

Intermediates of general formula XIII wherein $R^4$ represents the cyano group may also be prepared by reacting the corresponding carboxylic acid with a chlorinating agent, preferably thionyl chloride at ambient to reflux temperature, followed by reaction of the intermediate acid chloride with ammonia to give an intermediate amide which is then dehydrated by heating with a dehydrating reagent, preferably phosphorus oxychloride at a temperature from 50-250°C.

Intermediate carboxylic acids above may be prepared by hydrolysis of the corresponding esters preferably using a base such as sodium hydroxide and a solvent such as aqueous alcohol, and at a temperature from 0°C to the reflux temperature of the reaction mixture.

Compounds of general formula IX can be prepared by methods known per se.

The following Examples and Reference Examples illustrate the preparation of compounds of general formula I according to the present invention:-

[Chromatography was effected on a silica column (Merck 0.040-0.063 mm)at a pressure of 6.8$Nm^{-2}$ unless otherwise stated.]

## EXAMPLE 1

Compounds 1, 2 and 3.

Fuming nitric acid (1.7ml) was added dropwise to a stirred solution of 5-acetamido-3-bromo-1-(2,6-dichloro-4-trifluoromethylphenyl)-pyrazole (4.7g) and acetic anhydride (1ml) in glacial acetic acid (60ml). After 1.5h the reaction mixture was heated to 60°C and stirred at this temperature for 5h. The reaction mixture was cooled, poured into water (200ml), basified with saturated potassium carbonate solution (to pH 12) and extracted with diethyl ether. The extracts were washed with saturated sodium bicarbonate solution (2 x 100ml), dried and evaporated to give a brown gum (4.0g). This was purified by column chromatography using dichloromethane as eluent to give 5-acetamido-3-bromo-1-(2,6-dichloro-4-trifluoromethylphenyl)-4-nitropyrazole (2.9g), m.p. 179-180.5°C, in the form of colourless crystals.

By proceeding in a similar manner but replacing 5-acetamido-3-bromo-1-(2,6-dichloro-4-trifluoromethylphenyl)-pyrazole by the hereinafter indicated substituted pyrazole, there were obtained :-

5-Acetamido-3-chloro-1-(2,6-dichloro-4-trifluoromethylphenyl)-4-nitropyrazole, m.p. 177-178.5°C, in the form of colourless crystals, from 5-acetamido-3-chloro-1-(2,6-dichloro-4-trifluoromethylphenyl)-pyrazole.

5-Acetamido-3-cyano-1-(2,6-dichloro-4-trifluoromethylphenyl)-4-nitropropyrazole, m.p. 226-228.5°C, in the form of a cream-colored solid, from 5-acetamido-3-cyano-1-(2,6-dichloro-4-trifluoromethylphenyl)-pyrazole.

## EXAMPLE 2

Compounds 4, 5 and 6

A mixture of 5-acetamido-3-bromo-1-(2,6-dichloro-4-trifluoromethylphenyl)-4-nitropyrazole (1.0g), hydrobromic acid (48%; 5ml) and dioxan (15ml) was boiled under reflux for 18h. The reaction mixture was cooled and evaporated in vacuo and the residue treated with water to precipitate a solid which was filtered off and dried at 100°C to give 5-amino-3-bromo-1-(2,6-dichloro-4-trifluoromethylphenyl)-4-nitropyrazole (0.8g), m.p. 235-236°C, in the form of a colorless solid.

By proceeding in a similar manner but replacing 5-acetamido-3-bromo-1-(2,6-dichloro-4-trifluoromethylphenyl)-4-nitropyrazole by the hereinafter indicated substituted pyrazole and replacing hydrobromic acid by 6M hydrochloric acid, there were obtained :-

5-amino-3-chloro-1-(2,6-dichloro-4-trifluoromethylphenyl)-4-nitropyrazole, m.p. 219-220.5°C, in the form of colorless crystals from 5-acetamido-3-chloro-1-(2,6-dichloro-4-trifluoromethylphenyl)-4-nitropyrazole after purification by chromatography using dichloro methane as eluant followed by recrystallisation from dichloromethane-hexane.

5-Amino-3-cyano-1-(2,6-dichloro-4-trifluoromethylphenyl)-4-nitropyrazole, m.p. 255.5-256.5°C, in the form of a cream-coloured solid, from 5-acetamido-3-cyano-1-(2,6-dichloro-4-trifluoromethylphenyl)-4-nitropyrazole after purification by recrystallisation from toluene.

## EXAMPLE 3

Coumpound 7

Tertiary butyl nitrite (6.6ml) was added at room temperature to a solution of 5-amino-3-cyano-1-(2,6-dichloro-4-trifluoromethylphenyl)-4-nitropyrazole (3.7g), bromoform (13.3ml) and acetonitrile (10ml). The mixture was heated at 50-60°C for 4.5 hours and the solvents were evaporated to give an orange oil. This was purified by medium pressure liquid chromatography (eluant hexane/ether 2:1) to give 5-bromo-3-cyano-1-(2,6-dichloro-4-trifluoromethylphenyl)-4-nitropyrazole (3.4g) as a solid, m.p. 147-150°C.

## REFERENCE EXAMPLE 1

Pyridine (4.0ml) was added dropwise to a stirred solution of 5-amino-3-bromo-1-(2,6-dichloro-4-trifluoromethylphenyl)-pyrazole (5.0g), acetyl chloride (3.0g) and dry chloroform (30ml), causing an exothermic reaction (maximum temperature 60°C). The reaction mixture was stirred at laboratory temperature for 2h and evaporated to dryness. The residue was dissolved in ethanol (25ml), ammonia solution (d. 0.880; 25ml) was added, and the solution was boiled under reflux for 1h. The cooled reaction mixture was evaporated to dryness, dissolved in dichloromethane (100ml), washed with dilute hydrochloric acid (3 x 50ml) and water (50ml), dried over anhydrous magnesium sulphate, filtered, and evaporated to give 5-acetamido-3-bromo--1-(2,6-dichloro-4-trifluoromethylphenyl)-pyrazole (5.3g), m.p. 180-187°C, in the form of a pale brown solid.

By proceeding in a similar manner but replacing 5-amino-3-bromo-1-(2,6-dichloro-4-trifluoromethylphenyl)-pyrazole by the hereinafter indicated substituted pyrazole, there were obtained :-

5-Acetamido-3-chloro-1-(2,6-dichloro-4-trifluoromethylphenyl)-pyrazole, m.p. 195-197°C, in the form of a colourless solid after chromatography using dichloromethane as eluant from 5-amino-3-chloro-1-(2,6-dichloro-4-trifluoromethylphenyl)-pyrazole.

5-Acetamido-3-cyano-1-(2,6-dichloro-4-trifluoromethylphenyl)-pyrazole, m.p. 200.5-201.5°C, in the form of an orange solid, from 5-amino-3-cyano-1-(2,6-dichloro-4-trifluoromethylphenyl)-pyrazole.

## REFERENCE EXAMPLE 2

A mixture of 5-amino-4-carboethoxy-3-chloro-1-(2,6-dichloro-4-trifluoromethylphenyl)-pyrazole (3.3g), hydrobromic acid (45%; 30ml) and acetic acid (50ml) was boiled under reflux for 18h. The mixture was evaporated to low bulk and basified with sodium hydroxide solution (2M), and the product was filtered off, dried (2.9g) and recrystallised from a mixture of ethanol and water to give 5-amino-3-bromo-1-(2,6-dichloro-4-trifluoromethylphenyl)-pyrazole (2.5g), m.p. 132.5-134°C in the form of a colourless solid.

## REFERENCE EXAMPLE 3

Tertiary butyl nitrite (15.0g) was added dropwise to a stirred mixture of 4-carboethoxy-3,5-diamino-1-(2,6-dichloro-4-trifluoromethylphenyl)-pyrazole (50.0g), copper (II) chloride (21.0g) and acetonitrile (600ml) over a period of 10 min, with the temperature kept at 0°C by external cooling. The reaction mixture was stirred at this temperature for 2h and for a further 2h at laboratory temperature, evaporated to low bulk, and poured into hydrochloric acid (5M; 1.51). The resultant solution was extracted with dichloromethane (3 x 100ml) and the extracts were washed with hydrochloric acid (2M; 2 x 600ml), dried over magnesium sulphate, filtered and

evaporated to give a brown tar. The material was purified by dry-column chromatography on a silica gel column with dichloromethane:hexane (4:1) as eluent, followed by medium-pressure chromatography with the same solvents to give 5-amino-4-carboethoxy-3-chloro-1-(2,6-dichloro-4-trifluoromethylphenyl)-pyrazole (15.8g), m.p. 143-146.5°C, in the form of an orange solid.

## REFERENCE EXAMPLE 4

A mixture of 5-amino-4-carboethoxy-3-chloro-1-(2,6-dichloro-4-trifluoromethylphenyl)-pyrazole (5.0g), hydrochloric acid (6M; 75ml) and acetic acid (75ml) was boiled under reflux for 24h. The mixture was evaporated to low bulk and basified to pH 12 with sodium hydroxide solution (2M), and the product was extracted with diethyl ether (3 x 75ml). The combined extracts were evaporated to give a yellow, gummy solid (3.5g). This was dissolved in a mixture of hydrochloric acid (6M; 30ml) and dioxan (60ml) and boiled under reflux for 48h. Volatile materials were removed in vacuo and the residue was purified by chromatography with dichloromethane:hexane (4:1) as eluent to give 5-amino-3-chloro-1-(2,6-dichloro-4-trifluoromethylphenyl)-pyrazole (1.3g), m.p. 128-129°C in the form of an off-white solid.

## REFERENCE EXAMPLE 5

Ethyl dicyanoacetate potassium salt (35.2g) was added to a stirred suspension of 2,6-dichloro-4-trifluoromethylphenylhydrazine (49g) in hydrochloric acid (0.9M; 220ml) and the mixture was stirred and boiled under reflux for 18h, then cooled to precipitate a solid which was filtered off, triturated with diethyl ether (250ml) and dried to give an off-white solid which was recrystallised from ethyl acetate-hexane to give 4-carboethoxy-3,5-diamino-1-(2,6-dichloro-4-trifluoromethylphenyl)-pyrazole (29.2g), m.p. 196-197°C, in the form of an off-white solid.

## REFERENCE EXAMPLE 6

Sodium nitrite (8.8g) was added slowly to stirred concentrated sulphuric acid (90ml) and the resultant solution, when cool, was added dropwise, over a period of 20 min, to a solution of 2,6-dichloro-4-trifluoromethylaniline (23.0g) in acetic acid (180ml) with the temperature kept below 30°C. The mixture was then cooled to 5°C and added dropwise over 35min. to a stirred solution of ethyl 2,3-dicyanopropanoate (17.1g), sodium acetate (300g), water (1.2l) and ethanol (1.5ml), with the temperature maintained at 17-22°C. The mixture was stirred at room temperature for 2.5h, left overnight, diluted with water (2.0l) and extracted with dichloromethane (3 x 500ml). The combined extracts were washed three times with dilute sodium hydroxide solution and once with brine, dried over anhydrous magnesium sulphate and evaporated in vacuo to give a brown oil (25.2g). The residue was dissolved in a minimum amount of a hot toluene-hexane mixture and treated with charcoal. The solution was filtered and on cooling it gave 5-amino-3-cyano-1-(2,6-dichloro-4-trifluoromethylphenyl)-pyrazole (8.7g), m.p. 141-142°C, in the form of a pale brown solid.

## REFERENCE EXAMPLE 7

A solution of ethyl chloroformate (520g), malononitrile (330g) and tetrahydrofuran (500ml) was added dropwise over 1h to a stirred solution of potassium hydroxide (560g) and water (2.0l) at a temperature kept below 40°C by external ice-cooling. The reaction mixture was stirred at laboratory temperature for 1h, then cooled to 0°C to precipitate a solid which was filtered off and dried over phosphorus pentoxide to give ethyl dicyanoacetate potassium salt (334.4g) in the form of an off-white solid.

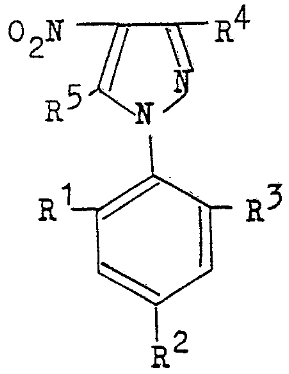

I

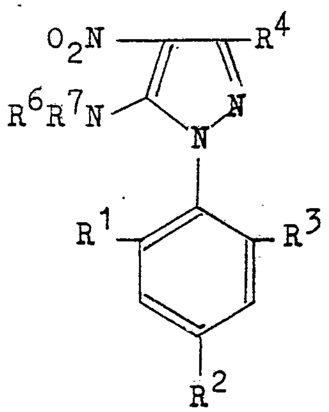

IB

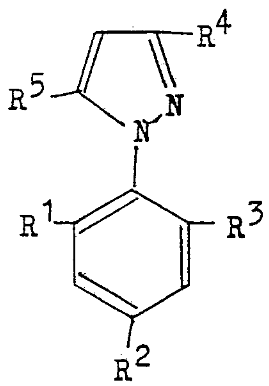

II

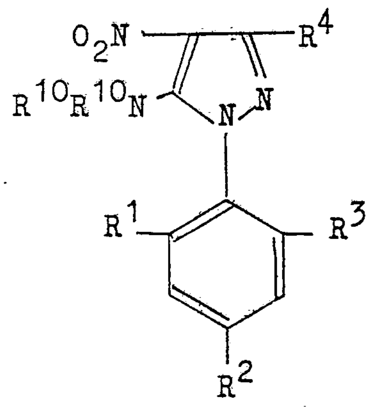

V

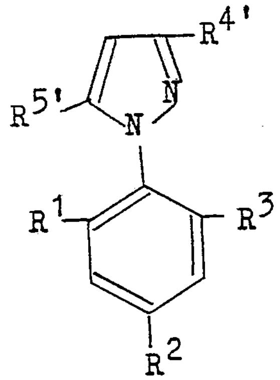

VIII

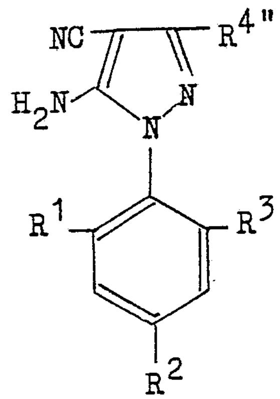

XI

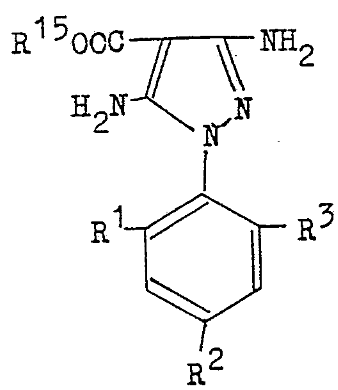

XII

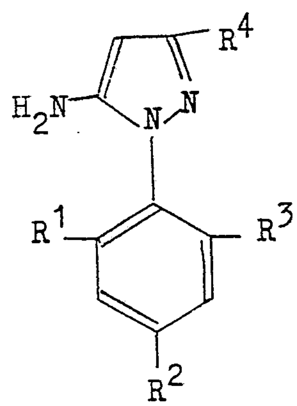

XIII

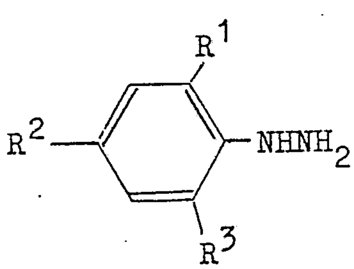

IX

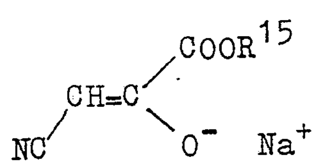

X

## Claims

1. A 4-nitro-N-phenylpyrazole derivative of the general formula:

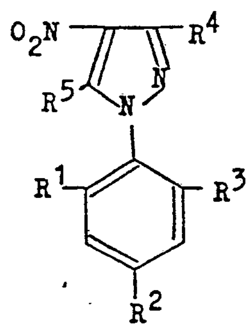

I

wherein $R^1$ represents a fluorine, chlorine or bromine atom, $R^2$ represents a chlorine or bromine atom, or a straight- or branched-chain alkyl or alkoxy group containing from 1 to 4 carbon atoms which may be unsubstituted or substituted by one or more halogen atoms, $R^3$ is as defined for $R^1$ or represents the hydrogen atom, $R^4$ represents a halogen atom or the cyano or nitro group, $R^5$ represents the hydrogen atom, or the amino group $-NR^6R^7$ wherein $R^6$ and $R^7$, which may be the same or different, each represents the hydrogen atom or a straight- or branched-chain alkyl group containing from 1 to 6 carbon atoms, the formyl group, a straight- or branched-chain alkanoyl group containing from 2 to 7 carbon atoms (or $R^6$ and $R^7$ together form a 5 or 6 membered cyclic imide with the nitrogen atom to which they are attached) which may be unsubstituted or substituted by one or more halogen atoms, or a straight- or branched-chain alkoxycarbonyl group containing from 2 to 7 carbon atoms, which may be unsubstituted or substituted by one or more halogen atoms, or $R^5$ represents a straight- or branched-chain alkoxymethyleneamino group containing from 2 to 5 carbon atoms which may be unsubstituted or substituted on methylene by a straight- or branched-chain alkyl group containing from 1 to 4 carbon atoms, or represents a fluorine, chlorine, bromine or iodine atom.

2. A compound according to claim 1 wherein $R^2$ represents a trifluoromethyl or trifluoromethoxy group.

3. A compound according to claim 1 wherein $R^1$, $R^2$ and $R^3$ together represent 2,4,6-trichloro, 2,6-dichloro-4-trifluoromethyl or 2,6-dichloro-4-trifluoromethoxy substitution of the phenyl group.

4. A compound according to claim 1 or 2 wherein $R^5$ represents the hydrogen atom or $-NR^6R^7$ and $R^1$, $R^2$ and $R^3$ together represent 2,6-dichloro-4-trifluoromethyl or 2,6-dichloro-4-trifluoromethoxy substitution of the phenyl group.

5. A compound according to any one of the preceding claims wherein $R^5$ represents an amino or acetamido group.

6. A compound according to claim 1 which is 5-acetamido-3-bromo-1-(2,6-dichloro-4-trifluoromethylphenyl)-4-nitropyrazole, 5-acetamido-3-chloro-1-(2,6-dichloro-4-trifluoromethylphenyl)-4-nitropyrazole, 5-acetamido-3-cyano-1-(2,6-dichloro-4-trifluoromethylphenyl)-4-nitropyrazole, 5-amino-3-bromo-1-(2,6-dichloro-4-trifluoromethylphenyl)-4-nitropyrazole, 5-amino-3-chloro-1-(2,6-dichloro-4-trifluoromethylphenyl)-4-nitropyrazole, 5-amino-3-cyano-1-(2,6-dichloro-4-trifluoromethylphenyl)-4-nitropyrazole and 5-bromo-3-cyano-1-(2,6-dichloro-4-trifluoromethylphenyl)-4-nitropyrazole.

7. A process for the preparation of a compound according to claim 1 which comprises:

(A) when $R^5$ represents an amino group $-NHR^{7'}$, wherein $R^{7'}$ represents the hydrogen atom or a straight- or branched-chain alkyl group containing from 1 to 6 carbon atoms, the hydrolysis of a compound of general formula I wherein $R^5$ represents an amino group $-NR^{6'}R^{7''}$ wherein $R^{6'}$ represents the formyl group or a straight- or branched-chain alkanoyl group containing from 2 to 7 carbon atoms, which may be unsubstituted or substituted by one or more halogen atoms, or a straight- or branched-chain alkoxycarbonyl group containing from 2 to 7 carbon atoms which may be unsubstituted or substituted by one or more halogen atoms and $R^{7''}$ is as defined for $R^7$ in claim 1 except that $R^{6'}$ and $R^{7''}$ together do not form a 5 or 6 membered cyclic imide with the nitrogen atom to which they are attached;

(B) when $R^5$ is as defined in claim 1 but does not represent an unsubstituted amino or alkylamino group, the nitration of a compound of the general formula:

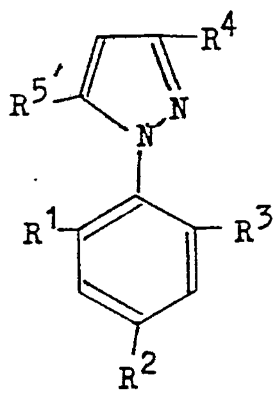

IIA

wherein $R^{5'}$ is as defined in claim 1 for $R^5$ but does not represent an unsubstituted amino or alkyl-amino group and $R^1$, $R^2$, $R^3$ and $R^4$ are as defined in claim 1 by treatment with a nitrating agent;

(C) the conversion by known methods of a compound of general formula I wherein $R^5$ represents a group $-NR^6R^7$ into a compound of general formula I wherein $R^5$ represents a different group $-NR^6R^7$; or, when $R^5$ in general formula I represents a straight- or branched-chain alkoxymethyleneamino group containing from 2 to 5 carbon atoms which may be unsubstituted or substituted on methylene by a straight- or branched-chain alkyl group containing from 1 to 4 carbon atoms, the reaction of a compound of general formula I wherein $R^5$ represents the unsubstituted amino group with a trisalkoxyalkane in the presence of an acidic catalyst; or

when $R^5$ represents a group $-NHCH_2R^{13}$ wherein $R^{13}$ represents a hydrogen atom or a straight- or branched-chain alkyl group containing from 1 to 4 carbon atoms, the reaction of a compound of general formula I wherein $R^5$ represents a group $-N{=}C(OR^{14})R^{13}$, wherein $R^{14}$ represents a straight- or branched-chain alkyl group containing from 1 to 4 carbon atoms, with a reducing agent;

(D) when $R^4$ in general formula I represents a fluorine, chlorine, bromine or iodine atom by the diazotisation of a compound corresponding to general formula II, wherein $R^4$ is replaced by the amino group and the hydrogen in the 4- position of the pyrazole ring is replaced by the nitro group, and conversion of the diazotised amino group by known methods into a fluorine, chlorine, bromine or iodine atom;

(E) when $R^5$ in general formula I represents a halogen atom by the diazotisation of a compound of general formula I wherein $R^5$ represents the amino group and conversion of the diazotised amino group by known methods into a halogen atom;

(F) when $R^5$ in general formula I represents a hydrogen atom, reaction of a compound of general formula I wherein $R^5$ represents the amino group with an alkyl nitrite in an inert organic solvent at ambient to reflux temperature; optionally followed by the conversion of a compound of general formula I thus obtained into another compound of general formula I.

8. An arthropodicidal, plant nematocidal, anthelmintic or anti-protozoal composition which comprises a compound of general formula I in association with one or more compatible diluents or carriers.

9. A method for the control of arthropod, plant nematode, helminth or protozoal pests at a locus which comprises treatment of the locus with an effective amount of a compound according to claim 1.

10. A compound of general formula I as defined in claim 1 for use in the manufacture of a medicament for the treatment of an arthropod, helminth or protozoal infection.

European Patent Office

# PARTIAL EUROPEAN SEARCH REPORT

which under Rule 45 of the European Patent Convention shall be considered, for the purposes of subsequent proceedings, as the European search report

Application number: EP 88305307.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | DE - A1 - 3 501 323 (BAYER AG)<br>* Claims 1,4,9,10; example 1 *<br>-- | 1,7 | C 07 D 231/16<br>C 07 D 231/36<br>C 07 D 231/40<br>C 07 D 403/04<br>A 01 N 43/56 |
| A | DE - A1 - 3 543 035 (BAYER AG)<br>* Claim 1 *<br>-- | 1,7 | |
| A | EP - A2/A3 - 0 224 831 (BAYER AG)<br>* Claims 1,3 *<br>---- | 1,7 | |

TECHNICAL FIELDS SEARCHED (Int. Cl.4)

C 07 D 231/00
C 07 D 403/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-8,10
Claims searched incompletely: -
Claims not searched: 9
Reason for the limitation of the search:

(Method for the treatment of a human or animal body by therapy; Article 52 (4) EPC)

| Place of search | Date of completion of the search | Examiner |
|---|---|---|
| VIENNA | 26-09-1988 | BRUS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1. 03.82